# EUROPEAN PATENT SPECIFICATION

(11) **EP 1 628 652 B1**
(45) Date of publication and mention of the grant of the patent: **11.07.2012**
(21) Application number: 04761006.8
(22) Date of filing: 13.05.2004
(51) Int. Cl.: A61K 31/343, A61K 31/165, A61K 31/138, A61K 31/4525, A61P 25/24

(54) **COMBINATION OF THE ANALEPTIC MODAFINIL AND AN ANTIDEPRESSANT FOR THE TREATMENT OF DEPRESSION**
KOMBINATION DES ANALEPTIKUM MODAFINIL MIT EINEM ANTIDEPRESSIVUM ZUR BEHANDLUNG VON DEPRESSION
COMBINAISON DE l'analeptique MODAFINIL ET D'un ANTIDEPRESSeur POUR TRAITER LA DEPRESSION

(30) Priority: 13.05.2003 US 469943 P; 12.05.2004 US 844187
(43) Date of publication of application: 01.03.2006
(73) Proprietor: CEPHALON, INC., West Chester, PA 19380 (US)
(72) Inventor: HASSMAN, Howard, Moorestown, NJ 08057 (US); HUGHES, Rodney, J., Kennett Square, PA 19348 (US)
(74) Representative: Hallybone, Huw George
(86) International application number: PCT/US2004/015194
(87) International publication number: WO 2004/100937

(56) References cited:
- WO-A-01/13921
- DEBATTISTA C ET AL: "A Prospective Trial of Modafinil as an Adjunctive Treatment of Major Depression" JOURNAL OF CLINICAL PSYCHOPHARMACOLOGY 2004 UNITED STATES, vol. 24, no. 1, 2004, pages 87-90, XP009035457 ISSN: 0271-0749
- NINAN PHILIP T ET AL: "Adjunctive modafinil at initiation of treatment with a selective serotonin reuptake inhibitor enhances the degree and onset of therapeutic effects in patients with major depressive disorder and fatigue." THE JOURNAL OF CLINICAL PSYCHIATRY. MAR 2004, vol. 65, no. 3, March 2004 (2004-03), pages 414-420, XP009035454 ISSN: 0160-6689
- MENZA M A ET AL: "Modafinil augmentation of antidepressant treatment in depression" JOURNAL OF CLINICAL PSYCHIATRY 2000 UNITED STATES, vol. 61, no. 5, 2000, pages 378-381, XP009035452 ISSN: 0160-6689
- KOGEORGOS J (REPRINT) ET AL: "Modafinil as augmentation of antidepressant therapy" EUROPEAN NEUROPSYCHOPHARMACOLOGY, (OCT 2002) VOL. 12, SUPP. [3], PP. S211-S212. PUBLISHER: ELSEVIER SCIENCE BV, PO BOX 211, 1000 AE AMSTERDAM, NETHERLANDS. ISSN: 0924-977X., vol. 12, no. S3, October 2002 (2002-10), XP002293250
- MARKOVITZ P J ET AL: "An open-label trial of modafinil augmentation in patients with partial response to antidepressant therapy [2]" JOURNAL OF CLINICAL PSYCHOPHARMACOLOGY 2003 UNITED STATES, vol. 23, no. 2, April 2003 (2003-04), pages 207-209, XP009035470 ISSN: 0271-0749
- HOLDER G ET AL: "Reduction of daytime sleepiness in a depressive patient during adjunct treatment with modafinil" JOURNAL OF PSYCHIATRIC RESEARCH 2002 UNITED KINGDOM, vol. 36, no. 1, 2002, pages 49-52, XP002293252 ISSN: 0022-3956
- SCHILLERSTROM JASON E ET AL: "Modafinil augmentation of mirtazapine in a failure-to-thrive geriatric inpatient." INTERNATIONAL JOURNAL OF PSYCHIATRY IN MEDICINE. 2002, vol. 32, no. 4, 2002, pages 405-410, XP009035480 ISSN: 0091-2174
- SCHWARTZ THOMAS L ET AL: "Modafinil in the treatment of depression with severe comorbid medical illness." PSYCHOSOMATICS. 2002 JUL-AUG, vol. 43, no. 4, July 2002 (2002-07), pages 336-337, XP009035497 ISSN: 0033-3182
- KAUFMAN K R ET AL: "Modafinil monotherapy in depression" EUROPEAN PSYCHIATRY 2002 FRANCE, vol. 17, no. 3, 2002, pages 167-169, XP002293253 ISSN: 0924-9338

## Description

### BACKGROUND OF THE INVENTION

### 1. Modafinil

Modafinil, C₁₅H₁₅NO₂S, also known as 2-(benzhydrylsulfinyl) acetamide, or 2-[(diphenylmethyl) sulfinyl] acetanide, is a synthetic acetamide derivative with wake-promoting activity, the structure of which has been described in French Patent No. 78 05 510 and in U.S. Patent No. 4,177,290 ('290), and which has been approved by the United States Food and Drug Administration for use in the treatment of excessive daytime sleepiness associated with narcolepsy. A method of preparation of a racemic mixture is described in the '290 patent and a method of preparation of a levorotatory isomer is described in U.S. Patent No. 4,927,855. The levorotatory isomer is reported to be useful for treatment of hypersomnia, depression, Alzheimer's disease and to have activity towards the symptoms of dementia and loss of memory, especially in the elderly.

The primary pharmacological activity of modafinil is to promote wakefulness. Modafinil promotes wakefulness in rats (Touret et al., 1995; Edgar and Seidel, 1997), cats (Lin et al., 1992), canines (Shelton et al., 1995) and non-human primates (Hemant et al, 1991) as well as in models mimicking clinical situations, such as sleep apnea (English bulldog sleep disordered breathing model) (Panckeri et al, 1996) and narcolepsy (narcoleptic canine) (Shelton et al, 1995).

Modafinil has also been described as an agent with activity in the central nervous system, and as a useful agent in the treatment of Parkinson's disease (U.S. Patent No. 5,180,745); in the protection of cerebral tissue from ischemia (U.S. Patent No. 5,391,576); in the treatment of urinary and fecal incontinence (U.S. Patent No. 5,401,776); and in the treatment of sleep apneas and disorders of central origin (U.S. Patent No. 5,612,379). U.S. Patent No. 5,618,845 describes modafinil preparations of a defined particle size less than about 200 microns. In addition, modafinil may be used in the treatment of eating disorders, or to promote weight gain or stimulate appetite in humans or animals (U.S. Patent No. 6,455,588), or in the treatment of attention deficit hyperactivity disorder (ADHD) (U.S. Patent No. 6,346,548), or fatigue, especially fatigue associated with multiple sclerosis (U.S. Patent No. 6,488,164).

Modafinil has been shown to be effective in treating narcolepsy, sleepiness, excessive sleepiness (e.g., sleepiness associated with disorders of sleep and wakefulness), excessive daytime sleepiness associated with narcolepsy, Parkinson's disease, urinary incontinence, multiple sclerosis fatigue, ADHD, Alzheimer's disorder, sleep apnea, obstructive sleep apnea, depression, and ischemia.

Narcolepsy is a chronic disorder characterized by intermittent sleep attacks, persistent, excessive daytime sleepiness and abnormal rapid eye movement ("REM") sleep manifestations, such as sleep-onset REM periods, cataplexy, sleep paralysis and hypnagogic hallucinations, or both. Most patients with narcolepsy also have disrupted nocturnal sleep. Pathological somnolence, whether due to narcolepsy or other causes, is disabling and potentially dangerous. Causes of pathological somnolence, other than narcolepsy, include chronic sleep loss; sleep apnea; and other sleep disorders. Whether due to narcolepsy or other causes, pathological somnolence produces episodes of unintended sleep, reduced attention, and performance errors. Consequently, it is linked to a variety of transportation and industrial accidents. A therapeutic agent that reduces or eliminates pathological somnolence would have important implications not only for individual patients, but also for public health and safety.

Other uses of modafinil have been presented. U.S. Pat. No. 5,180,745 discloses the use of modafinil for providing a neuroprotective effect in humans, and in particular for the therapy of Parkinson's disease. The levorotatory form of modafmil, i.e., (-) benzhydrylsulfinyl-acetamide, may have potential benefit for therapy of depression, hypersomnia and Alzheimer's disease (U.S. Pat. No. 4,927,855). European Published Application 547952 discloses the use of modafinil as an anti-ischemic agent. European Published Application 594507 discloses the use of modafinil to treat urinary incontinence.

U.S. Pat. No. RE37,516 discloses pharmaceutical compositions having a defined particle size, and in particular compositions wherein 95% of the cumulative total of the effective amount of modafinil particles in the composition have a diameter less than about 200 microns.

### 2. Antidepressants

Antidepressants, including selective serotonin reuptake inhibitors (SSRIs) have become first choice therapeutics in the therapy of depression, certain forms of anxiety and social phobias. In some instances, SSRIs can be more favored because they are effective, well tolerated and have a favorable safety profile compared to the classic tricyclic antidepressants.

However, there can be problems associated with any anti-depressant. Current antidepressant therapy can exhibit a delayed onset and modest proportion in achieving response or remission. For example, the response at 6 weeks to the selective serotonin reuptake inhibitor (SSRI) fluoxetine is about 50%. Remission rates with SSRIs at 8 weeks are about 35%. Delayed, incomplete and lack of response of a major depressive disorder to antidepressant therapy can be problematic for numerous reasons, including premature treatment discontinuation. Sometimes symptoms even worsen during the first weeks of therapy. In other cases, non-compliance can be related to side effects, including sexual dysfunction.

Fatigue and excessive sleepiness are among the symptoms of a major depressive disorder, and can be adverse experiences associated with antidepressant therapy and are often residual symptoms inadequately treated with SSRI antidepressant therapy.

In addition, patients sometimes suffer side effects associated with antidepressant therapy and withdrawal of antidepressant therapy.

Because residual symptoms to antidepressant therapy predisposes patients with depression to a greater risk of relapse and greater probability of recurrence, rapid achievement of remission is an important consideration in choosing the most appropriate treatment strategy.

New therapies that address one or more of these problems are needed.

### SUMMARY OF THE INVENTION

In one embodimeng the present invention includes a pharmaceutical composition for use in decreasing the onset time of an antidepressant in an animal subject. The use includes co-administering an effective amount of modafinil, with an antidepressant, wherein the patient has been free of antidepressant therapy for at least 4 weeks.

### BRIEF DESCRIPTION OF THE DRAWING

Figure 1A: Mean 21-item HAMD-21 total scores for baseline and weeks 1 through 6.
Figure 1B: Mean 31-item HAMD-31 total scores for baseline and weeks 1 through 6.
Figure 2: Percentages of patients with response and remission for baseline and weeks 1 through 6.

### DETAILED DESCRIPTION OF THE INVENTION

### 1. Analeptic Agents

Analeptics are drugs that principally act as or are used as a central nervous system stimulant.

The analeptic used in the practice of the invention is modafinil.

### 2. Antidepressants

Useful antidepressants include but are not limited to tricyclic antidepressants ("TCAs'), Selective Serotonia Reuptake Inhibitors ("SSRIs"), Serotonin and Noradrenaline Reuptake Inhibitors ("SMRIs"), Dopamine, Reuptake Inhibitors; ("DRIs'"), Noradrenaline Reuptake Inhibitors ("NRUs"), Dopamine, Serotonin and Noradreoaime Reuptake Inhibitors ("DSNRIs") and Monoamine Oxidase Inhibitors ("MAOIs) including reversible inhibitors of monoamine oxidase type A (RIMAs).

In certain embodiments, a suitable antidepressant can include, but is not limited to, one or more of the following antidepressants: adalanserin hydrochloride; adinazolam; adinazolam mesylate; alaptoclate; aletamine hydrochloride; amedalin hydrochloride; amitriptyline hydrochloride; amoxapine; aptazapine maleate; azaloxan fumarate; azepindole; azipramine hydrochloride; bipenarnol hydrochloride; bupropion hydrochloride; butacetin; butriptyline hydrochloride; caroxazone; cartazolate; ciclazindol; cidoxepin hydrochloride; cilobamine mesylate; citalipram; clodazon hydrochloride; clomipramine hydrochloride; cotinine fumarate; cyclindole; cypenamine hydrochloride; cyprolidol hydrochloride; cyproximide; daledalin tosylate; dapoxetine hydrochloride; dazadrol maleate; dazepinil hydrochloride; desipramine hydrochloride; dexamisole; deximafen; dibenzepin hydrochloride; dioxadrol hydrochloride; dothiepin hydrochloride; doxepin hydrochloride; duloxetine hydrochloride; eclanamine maleate; encyprate; etoperidone hydrochloride; fantridone hydrochloride; fehmetozole hydrochloride; fenmetramide; fezolamine fumarate; fluotracen hydrochloride; fluoxetine; fluoxetine hydrochloride; fluparoxan hydrochloride; gamfexine; guanoxyfen sulfate; imafen hydrochloride; imiloxan hydrochloride; imipramine hydrochloride; indeloxazine hydrochloride; intriptyline hydrochloride; iprindole; isocarboxazid; ketipramine fumarate; lofepramine hydrochloride; lortalamine; maprotiline; maprotiline hydrochloride; melitracen hydrochloride; milacemide hydrochloride; minaprine hydrochloride; mirtazapine; moclobemide; modaline sulfate; napactadine hydrochloride; napamezole hydrochloride; nefazodone hydrochloride; nisoxetine; nitrafudamhydrochloride; nomifensine maleate; nortriptyline hydrochloride; octriptyline phosphate; opipramol hydrochloride; oxaprotiline hydrochloride; oxypertine; paroxetine; phenelzine sulfate; pirandamine hydrochloride; pizotyline; pridefine hydrochloride; prolintane hydrochloride; protriptyline hydrochloride; quipazine maleate; rolicyprine; seproxetine hydrochloride; sertraline hydrochloride; sibutramine hydrochloride; sulpiride; suritozole; tametraline hydrochloride; tampramine fumarate; tandamine hydrochloride; thiazesim hydrochloride; thozalinone; tomoxetine hydrochloride; trazodone hydrochloride; trebenzomine hydrochloride; trimipramine; trimipramine maleate; venlafaxine hydrochloride; viloxazine hydrochloride; zimeldine hydrochloride; zometapine.

In certain embodiments, the antidepressant includes citalipram, fluoxetine, fluoxetine hydrochloride, paroxetine, paroxetine hydrochloride, and/or clomipramine hydrochloride, with citalipram, paroxetine, fluoxetine and fluoxetine hydrochloride preferred, with citalipram most preferred.

Other drugs which are useful in treating depressive disorders, e.g., tiagabine, can also be used in the practice of the invention.

### 3. Variants, Analogs, Salts, Different Forms

Antidepressants not listed above, including but not limited to structural analogs of the above compounds, that are safe and effective, are also useful in the practice of the invention.

Included within the scope of this invention are the various individual stereoisomers, including diastereomers and enantiomers (e.g., the L and/or R-isomer of modafinil) as well as mixtures thereof. In addition, compounds useful in this invention also include any pharmaceutically acceptable salts, for example: alkali metal salts, such as sodium and potassium; ammonium salts; monoalkylammonium salts; dialkylammonium salts; trialkylammonium salts; tetraalkylammonium salts; and tromethamine salts. Hydrates, solvates, and polymorphs of the compounds described above are included within the scope of this invention. Combinations of analeptics and of antidepressants can also be employed. The compounds can be substantially pure or mixed with other ingredients.

### 4. Depressive Disorders

The invention is useful in the treatment of depression, including mild to severe or acute depression, that may be caused by any of a number of factors, including, for example, depression associated with alcohol or drug abuse. The invention is also useful in the treatment of other disorders for which antidepressants are sometimes prescribed. These include, for example, anxiety, stress, social phobia, panic, obsession, compulsive behavior, pain (e.g., neuropathic and inflammatory pain) etc. Such disorders, for which antidepressants have been shown to have clinically beneficial effects, are herein referred to collectively as "depressive disorders."

### 5. Therapeutically Effective Amounts of Analeptics and Antidepressants

In one embodiment of the present invention, an amount of modafinil, administered to a patient can include 5, 10, 15, 20, 30, 40, 50, 60, 70, 75, 80, 90, 100, 200, 300 and/or 400 mg. of modafinil, or combinations thereof. Typically, modafinil can be administered in 50, 75, 100 and 200 mg. amounts. However, when used in combination with one or more antidepressants, as described herein, the amount of modafinil necessary to alleviate all or a portion of the symptoms associated with antidepressant therapy can be reduced. Accordingly, one embodiment of the present invention includes 100 mg. or less of modafinil when administered with an antidepressant, either as a combined unit dose with the antidepressant or as a separate dose, A single unit dose containing both modafinil and an antidepressant is a preferred composition of the present invention, as described below.

Typically, one or more antidepressants can be administered in the amounts known to be effective for each antidepressant. More specifically, in the present invention, an antidepressant can be administered in an amount effective to alter the depressive state of an animal subject, i.e., the amount of antidepressant that would be administered to the animal subject if the antidepressant was administered alone. Suitable amounts can include 5,10, 15, 20, 30, 40, 50, 60, 70, 80, 90, 100, 200, 300 and/or 400 mg. of a particular antidepressant, and combinations thereof However, in the present invention, when used in combination with an analeptic, i.e modafinil, the overall amount of an administered antidepressant can be reduced by 10%, 20%, 30%, 40%, 50%, 60%, 70% or 80%, while still providing an antidepressant effect Accordingly, one embodiment of the present invention includes administering less than an amount of antidepressant relative to the amount of antidepressant administered to an animal subject if administered alone.

Generally, for daily oral doses of active compounds, the combined total of analeptic and one or more antidepressants will be from about 0.01 mg/kg per day to about 2000 mg/kg per day. It is elected that IV doses in the range of about 1 to 1000 mg/cm³ per day will be effective.

In some embodiments of the present invention, the respective weight ratio of analeptic to antidepressant can be from 0.01: 1 to 1:1 to 100:1, possibly 1000:1 In some embodiments the weight ratio can be 1:1 to 7:1 or 10:1, most preferably 1:1 to 5:1.

A dosage form containing an above described amount of an analeptic (i.e, modafinil) and one or more antidepressants can provide to a patient improved fatigue symptoms, as well as improve waking functioning, as demonstrated by the effects of fatigue, energy, alertness and cognitive function (e.g. psychomotor retardation).

### 6. Preparation of a Composition of the Present Invention

To prepare a pharmaceutical composition of this invention, an analeptic, i.e, modafinil, and an antidepressant, including but not limited to one or more of the antidepressants described above, can be intimately admixed. The mixture can further optionally include a pharmaceutical canter according to conventional pharmaceutical compounding techniques, which carrier may take a wide variety of forms depending on the form of preparation desired for administration, e.g., oral, by suppository, or parenteral. The amount of each active component in the composition can correspond to the amounts described above. Pharmaceutically acceptable carriers include, e.g., stabilizers binders, fillers, disintegrants, lubricants, coatings, sweeteners, flavors, colors, diluents, etc. Such a composition, when used for the therapy of a depressive disorder preferably can include therapeutically effective amounts of an analeptic and antidepressant.

In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, and tablets, suitable carriers and additives include starches, sugars, diluents, granulating agents, lubricants, binder disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques.

For parentarals, the carrier will usually comprise sterile water, though other ingredients, for example, for purposes such as aiding solubility or for preservation, may be included. Injectable suspensions may also be prepared in which case appropriate liquid carriers, suspending agents and the like may be employed.

In one embodiment, a pharmaceutical composition of the present invention can be administered in a tablet or capsule form or other suitable unit dose form. A tablet or capsule of the present invention can contain one or more of the following inactive ingredients: lactose hydrous, pregelatinized starch, microcrystalline cellulose, sodium starch glycolate, magnesium stearate, purified water, carnauba wax, hydroxypropyl methylcellulose, titanium dioxide, polyethylene glycol, synthetic iron oxide, and polysorbate 80, etc.

Accordingly, a pharmaceutical compositions herein will contain, per dosage unit, e.g., tablet, capsule, powder injection, teaspoonful, suppository and the like from about 5 to about 1000 mg, or more, of an analeptic and antidepressant. In one embodiment of the invention, each single dosage unit (or unit dose) includes both an amount of an analeptic and an amount of an antidepressant. In such embodiment, it is not necessary that each single dosage unit include an effective amount so long as the total amount of drug administered to a patient is an effective amount of each. Therefore, for example, a patient may require 2 or more single dosage units to receive effective amounts of both agents.

When administered, the formulations of the invention are applied in pharmaceutically acceptable amounts and in pharmaceutically acceptable compositions. Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulfuric, nitric, phosphoric, maleic, acetic, salicylic, p-toluene sulfonic, tartaric, citric, methane sulfonic, formic, malonic, succinic, naphthalene-2-sulfonic, and benzene sulfonic. Also, pharmaceutically acceptable salts can be prepared as alkaline metal or alkaline earth salts, such as sodium, potassium or calcium salts.

Suitable buffering agents include: acetic acid and a salt (1-2% W/V); citric acid and a salt (1-3% W/V); boric acid and a salt (0.5-2.5% W/V); and phosphoric acid and a salt (0.8-2% W/V). Suitable preservatives include benzalkonium chloride (0.003-0.03% W/V); chlorobutanol (0.3-0.9% W/V); parabens (0.01-0.25% W/V) and thimerosal (0.004-0.02% W/V).

Dosage may be adjusted appropriately to achieve desired drug levels, locally or systemically. As noted above, generally, daily oral doses of active compounds will be from about 0.01 mg/kg per day to 2000 mg/kg per day. In the event that the response in a subject is insufficient at such doses, even higher doses (or effective higher doses by a different, more localized delivery route) may be employed to the extent that patient tolerance permits. Continuous IV dosing over, for example 24 hours or multiple doses per day is contemplated to achieve appropriate systemic levels of compounds.

A variety of administration routes are available. The particular mode selected will depend of course, upon the particular drug selected, the severity of the disease state(s) being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, sublingual, topical, nasal, transdermal or parenteral routes. The term "parenteral" includes subcutaneous, intravenous, intramuscular, or infusion.

The compositions may conveniently be presented in unit dosage form and may be prepared by any of the methods well known in the art of pharmacy. In general, the compositions are prepared by uniformly and intimately bringing the compounds into association with a liquid carrier, a finely divided solid carrier, or both, and then, if necessary, shaping the product.

Compositions suitable for oral administration may be presented as discrete units such as capsules, cachets, tablets, or lozenges, each containing a predetermined amount of the active compound. Other compositions include suspensions in aqueous liquors or non-aqueous liquids such as a syrup, an elixir, or an emulsion.

Other delivery systems can include time-release, delayed release or sustained release delivery systems. Such systems can avoid repeated administrations of the active compounds of the invention, increasing convenience to the subject and the physician. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. In addition, a pump-based hardware delivery system can be used, some of which are adapted for implantation.

Another embodiment of the present invention provides a kit or device which can facilitate the administration of an amount of an analeptic and an antidepressant to treat a depressive disorder. Specifically, a kit according to the present invention includes at least one dosage form containing an analeptic, i.e. modafinil, and a separate dosage form containing at least one antidepressant. One suitable kit of the present invention includes a blister pack having a unit dose of modafinil and a separate unit dose of an antidepressant. Most preferably, the unit dose of modafinil includes a 50, 75, 100 or 200 mg. tablet of modafinil and the unit dose of antidepressant includes a 10, 20, 30, 40 or 50 mg. tablet of antidepressant. The kit or device can also include instructions concerning administration of the analeptic and antidepressant. Preferably, the instructions provide administration guidance according to one or more of the administration schemes set forth below.

The analeptic and/or antidepressant can be in any suitable dosage form, including but not limited to solid dosage forms including tablets, capsules, pills, troches, cachets, and the like, and/or liquid dosage forms such as an oral elixir or an IV fluid. The dosage form of the analeptic can be the same type or a different type than the antidepressant.

In yet another embodiment, the present invention includes a transdermal drug delivery system ("'TDDS"). A TDDS suitable for use with the invention in patch form typically contains at least: (1) a backing layer and (2) a carrier formulated with an effective amount of an antidepressant and optionally modafinil.

Preferred patches include (1) the matrix type patch; (2) the reservoir type patch; (3) the multi-laminate drug-in-adhesive type patch; and (4) the monolithic drug-in-adhesive type patch; and (Ghosh, T. K.; Pfister, W. R.; Yum, S. I. Transdermal and Topical Drug Delivery Systems. Interpharm Press, Inc. p. 249-297. These patches are generally available commercially.

For practice of the invention, the matrix type and the drug-in-adhesive type patches are especially preferred. The more preferred drug-in-adhesive patch is the monolithic type.

Transdermal drug delivery systems other than standard patches can also be used. These include, for example, osmotic pump systems, ultrasonic systems, ointments, pastes, gels, medicated powders, creams, lotions, aerosols, sprays, foams, medicated adhesives and the like.

### 7. Method of Treatment/Therapy

### A. Administration Schemes and Timing of Treatment of an Analeptic (i.e modafinil) and Antidepressant

An analeptic (i.e.modafinil) and an antidepressant can be combined together into a single unit dose, but can also be administered separately as two or more distinct doses.

Thus, in some embodiments of the invention, a treatment of a disorder related to depression can be through the use of separate dosage forms - one or more analeptic doses and one or more antidepressant doses. Accordingly, a dose of an analeptic can be administered at a different time relative to the antidepressant dose or simultaneously (i.e., analeptic dose administration within less than 1 hour before or after administration of the antidepressant). However, if simultaneous administration is desired, the administration of the analeptic and antidepressant can also be though the use of a single unit dose including both an analeptic and antidepressant.

In patients that are beginning antidepressant therapy, i.e. patients that are substantially free of antidepressants or patients that have been free of antidepressant therapy for 4 or more weeks, the dosage form containing the analeptic can be administered before and/or at about the same time as an initial administration of the antidepressant. In such an embodiment, one or more administrations of an analeptic can be within 72 hours, preferably within 48 hours, more preferably within 24 hours, most preferably within 1 hour or moments before an initial administration/dosing of an antidepressant. After the initial administration of the analeptic and antidepressant, subsequent dosings of the analeptic and antidepressant can continue at a typical rate, e.g., typically one or two 50, 75, 100 to 200 mg. doses of modafinil per day and 10, 20, 30, 40, 50 mg. of antidepressant per day. Further, after the initial administration of the antidepressant, the dosings of the analeptic and antidepressant can be in separate dosage forms or in a single unit dose. However, if a dose of an analeptic is to be administered before a subsequent dose of an antidepressant, separate dosage forms for each are preferred.

Additionally, in patients that are substantially free of antidepressants, the initial administration of the analeptic can coincide with or be nearly simultaneous with the initial administration of an antidepressant. This can be accomplished through the use of separate dosage forms of an analeptic and antidepressant which can then be administered together simultaneously (i.e., within 1 hour or less, before or after the antidepressant) or through the use of a single unit dose including both an analeptic and an antidepressant, as noted above.

In a further embodiment, initial administration of an analeptic to a patient can occur and/or continue after antidepressant therapy has ended. Preferably, this is accomplished by administering an amount of the analeptic to the patient and the administration of which can continue for 1, 2, 5, 10, 20, or 30 days, or more, after antidepressant therapy cessation.

In embodiments where the analeptic and antidepressant are in separate dosage forms, the administration of the analeptic can preferably occur within moments, or in less than 1 hour, or less than 5 hours, or less than 24 hours or less than 48 hours, or less than 72 hours before or less than 1 hour after administration of the antidepressant, unless otherwise indicated by a particular method of treatment below.

### B. Reduction of Onset Time of Antidepressant Effect

The time lapse between initiation of antidepressant therapy and alleviation of depressive symptoms can be shortened. In one embodiment of the present invention, depressive symptoms can be improved after the initiation of adiministration of an analeptic, i.e. modafinil, before or during antidepressant therapy or by following one or more of the timing schemes set forth above.

The time of improvement can be from 1, 2, 4, 7,10, and 14 days relative to antidepressant therapy alone.

In a further embodiment, the present invention includes a pharmaceutical composition for use in decreasing the onset time of an antidepressant in an animal subject. The use includes the step of co-administering an effective amount of analeptic, i.e. modafinil with an antidepressant. The amount of analeptic and duration of pretherapy can vary from subject to subject. However, it is preferred that the timing of administration of the analeptic follow one or more of the timing schemes set forth above.

In one embodiment, the amount of analeptic includes an effective amount of modafinil, typically from about 100 mg to about 200 mg of modafinil administered once or twice daily for a period of less than 2 days, preferably less than 10 days, prior to the initiation therapy of the antidepressant with which it is desired to have a decrease in onset time. In another embodiment, the first administration of an analeptic can be within 72 hours, preferably within 48 hours, more preferably within 24 hours, most preferably within 1 hour or within moments before initial administration of an antidepressant. As noted above, the administration of the analeptic can also optionally continue during antidepressant therapy.

The analeptic can be administered orally, nasally, rectally, intravenously, epidurally, intraperitoneally, subcutaneously, intramuscularly or intrathecally.

### DEFINITIONS

"Particle," as used herein, refers to an aggregated physical unit of the acetamide compound, i.e., a piece or a grain of acetamide.

As used herein, "about" means plus or minus ten percent of the indicated value, such that "about 20 mg" indicates 18 to 22 mg.

As used herein, "consisting essentially of" refers to excluding other active ingredients but including excipients and additional amounts of the active ingredient to account for degradation or otherwise.

An "effective amount," as used herein, is an amount of modafinil and/or antidepressant that is effective for treating a depressive state, i.e., an amount of modafinil and/or antidepressant that is able to reduce, alleviate or eliminate certain symptoms associated with depression and/or antidepression therapy.

A "pharmaceutical composition," as used herein, means a medicament for use in treating a mammal that comprises modafinil prepared in a manner that is appropriate for administration to a mammal. A pharmaceutical composition according to the invention may also, but does not of necessity, include a non-toxic pharmaceutically acceptable carrier. A pharmaceutical composition can also include bulk active modafinil for use in preparing dosage forms. A pharmaceutical composition can also include modafinil in combination with another active, preferably an antidepressant, more preferably an SSRI.

### Example

Eligible patients were previously diagnosed with MDD (single episode or recurrent), four patients had significant fatigue (Fatigue Severity Scale [FSS] score of greater than or equal to 4), and had not taken antidepressant therapy for greater than or equal to 4 weeks. Patients were evaluated at screening, baseline (shown in Table 1), and weeks 1, 2, 3, 4, 5, and 6.

| **Table 1. Baseline Patient Characteristics** | |
|---|---|
| | Modafinil + Fluoxetine or Paroxetine (N=29) |
| | |
| Mean age; years (SD) | 36.2 (8.6) |
| Mean weight; pounds (SD) | 173.1(57.5) |
| Gender; n (%) | |
| Female | 21 (72.4) |
| Race; n (%) | |
| ' Caucasian | 19 (65.5) |
| Mean years with disease (SD) | 2.7 (3.9) |
| Mean HAMD-21 score (SD) | 22.6 (4.9)* |
| Mean HAMD-31 score (SD) | 29.9 (7.4)* |
| Mean FSS score (SD) | 5.2 (0.8)* |
| Mean ESS score (SD) | 10.3 (4.9) |
| * N=28 | |
| ESS=Epworth Sleepiness Scale; FSS=Fatigue Severity Scale; HAMD=Hamilton Rating Scale for Depression; SD=standard deviation; VAS=Visual Analogue Scale | |

Patients were then started on a combination of an SSRI and modafinil.

Modafinil was initiated at 100 mg/day for 3 days and then titrated to 200 mg/day, depending on response and tolerability. SSRI therapy was either fluoxetine or paroxetine administered at 20 mg/day for 6 weeks.

### 1. Symptom Assessments

Depressive symptom changes were analyzed using HAMD-31, each of which were videotaped and rated independently, and HAMD-21 total score evaluations. HAMD-21 total score analyses were also performed to evaluate response and remission rates. Changes in fatigue were assessed using the FSS. A fatigue response was defined as an FSS score of less than 4 at any post-baseline visit. An FSS score of greater than or equal to 4 denotes pathologic levels of fatigue. Subjective sleepiness was assessed using the Epworth Sleepiness Scale (ESS). An ESS score of greater than or equal to 10 denotes pathologic levels of sleepiness. Symptoms associated with depression, including fatigue, mood, motivation, and concentration, were evaluated using patient-assessed Visual Analogue Scales (VAS).

### 2. Safety Monitoring

Safety was assessed by recording all reported adverse events by day of onset, type, severity, and relationship to study medication. Physical exams, vital signs, and clinical laboratory tests were conducted during the study.

### 3. Statistics

Continuous variables were analyzed using a paired t-test for normally distributed data or Wilcoxon signed rank test for non-normal data.

The numbers of responders (defined as a >50 % decrease in HAMD-21) and remitters (defined as a score of less than or equal to 7 in HAMD-21 at any post-baseline visit) were analyzed using the Wilcoxon signed rank test. Patients receiving at least 1 dose of a study drug were included in the safety analysis.

Descriptive statistics were used to summarize safety measures. Baseline characteristics of all patients are summarized in Table 1. Patients who received at least 1 dose of modafinil and had at least 1 post-baseline efficacy measurement were evaluated for efficacy (N=28). Twenty-nine patients were available for safety evaluation.

### 4. Treatment Outcomes

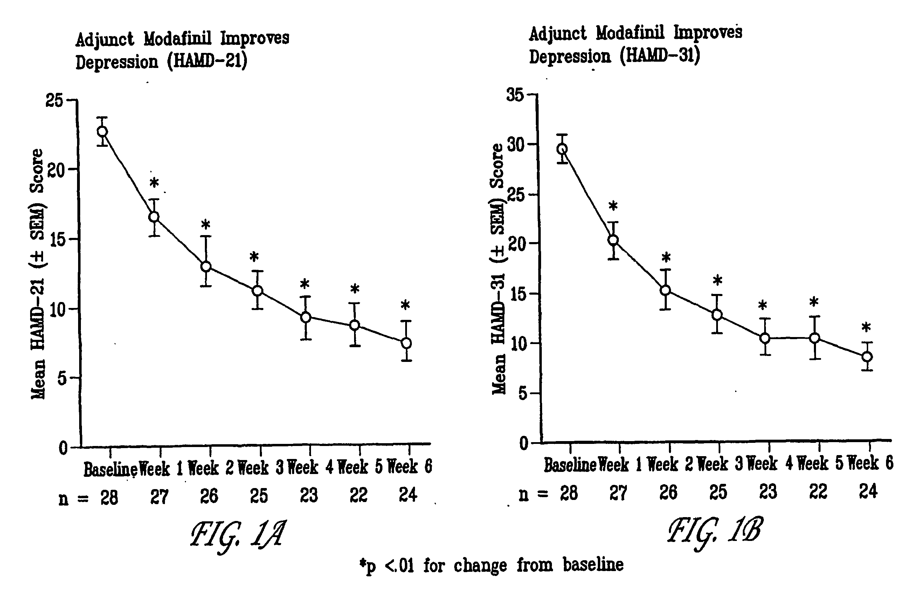

Modafinil combined with an SSRI significantly improved depression within 1 week of initiation, as shown by reductions from baseline in mean total HAMD-21 scores (Figure 1A). Statistically significant decreases in mean total HAMD-21 scores from baseline progressed to week 6. Modafinil combined with an SSRI significantly reduced mean total HAMD-31 scores from baseline within 1 week of initiation and progressed to week 6 (Figure 1B).

The average HAMD-31 score of the fourteen evaluable patients was 31.72 +/-7.28. Modafinil combined with fluoxetine or paroxetine significantly improved total HAMD-31 scores within 1 week of initiation (mean -9.47 +/-12.06; p<0.01). Improvement was maintained throughout the study (mean -23.06 +/- 13.55; p<0.01).

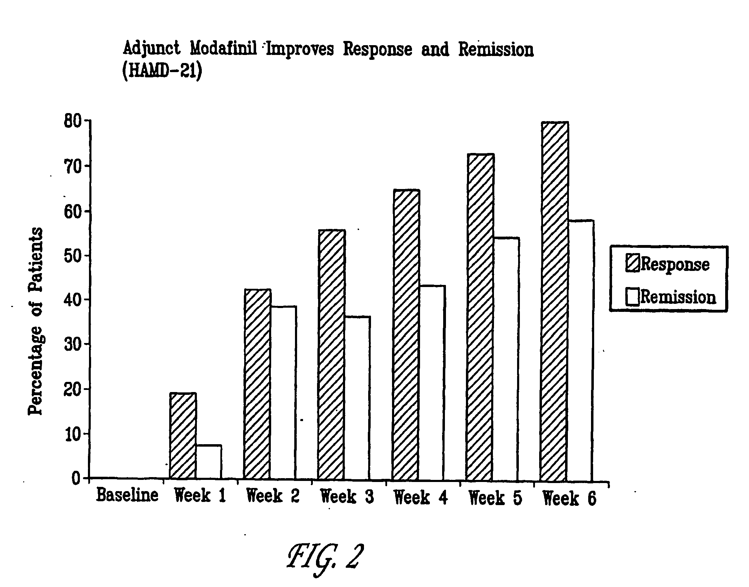

Response, defined as a greater than 50% decrease in baseline HAMD-21 score, was achieved by 42% of patients at week 2, 65% by week 4, and 79% at week 6, as shown in Figure 2. Remission of depressive symptoms, defined as less than or equal to 7 on HAMD-21, was achieved by 12% of patients at week 1, 39% of patients at week 2, 44% at week 4, and about 58% at week 6 (Figure 2).

### 5. Safety and Tolerability

Adjunct modafinil was well tolerated. Fifty-nine percent (17/29) of patients reported at least one adverse event. The most frequently reported adverse events were nausea (41 %) and headache (24%).

Adverse events were mild to moderate in severity, with no serious adverse events reported during the study. No clinically significant differences were found in vital signs, body weight changes, ECG, or laboratory parameters. Twenty-three of 29 patients (79%) completed the study. Three patients in the modafinil and fluoxetine group discontinued because of treatment-related adverse events: one reported agitation, anorexia, and headache; another reported headache and abnormal thinking; and a third reported insomnia, nausea, and nervousness. One patient was withdrawn due to protocol noncompliance. Two patients were lost to follow-up.

Based on the above, modafinil was found to be a rapid-acting and effective adjuvant medication in the treatment of residual symptoms in patients with depression and significant fatigue, and modafinil can provide a greater adjunctive effect when used in combination with SSRI therapy at initiation and the therapeutic strategy can result in a faster reduction of multiple dimensions of MDD symptoms.

## Claims

1. A pharmaceutical composition comprising modafinil and an antidepressant for use in treating a depressive disorder in a patient, wherein the patient has been free of antidepressant therapy for at least 4 weeks.

2. The pharmaceutical composition for the use according to claim 1, wherein the modafinil is the levorotatory isomer of modafinil.

3. The pharmaceutical composition for the use according to claim 1 or claim 2, wherein the antidepressant is selected from the group consisting of tricyclics, selective serotonin reuptake inhibitors, serotonin and noradrenaline reuptake inhibitors, monoamine oxidase inhibitors, and reversible inhibitors of monoamine oxidase type A.

4. The pharmaceutical composition for the use according to claim 3, wherein the antidepressant is citalopram, fluoxetine, fluoxetine hydrochloride, paroxetine, paroxetine hydrochloride, or clomipramine hydrochloride.

5. The pharmaceutical composition for the use according to claim 4, wherein the antidepressant is citalopram, fluoxetine or fluoxetine hydrochloride.

6. The pharmaceutical composition for the use according to claim 3, wherein the antidepressant is venlafaxine hydrochloride.

7. The pharmaceutical composition for the use according to any of claims 1-6, wherein the modafinil is administered in an amount of 50, 75, 100, or 200 mg per day.

8. The pharmaceutical composition for the use according to any of claims 1-6, wherein the modafinil is administered in an amount of 100 mg per day.

9. The pharmaceutical composition for the use according to any of claims 1-8, wherein the modafinil and the antidepressant are administered in a ratio of from 1:1 to 10:1, by weight.

10. The pharmaceutical composition for the use according to claim 9, wherein the modafinil and the antidepressant are administered in a ratio of from 1:1 to 7:1, by weight.

11. The pharmaceutical composition for the use according to claim 10, wherein the modafinil and the antidepressant are administered in a ratio of from 1:1 to 5:1, by weight.

12. The pharmaceutical composition for the use according to claim 7, wherein the antidepressant is administered in an amount of 10, 20, 30, 40, or 50 mg per day.

## Patentansprüche

1. Pharmazeutische Zusammensetzung, die Modafinil und ein Antidepressivum umfasst, zur Verwendung bei der Behandlung einer depressiven Störung bei einem Patienten, wobei der Patient mindestens 4 Wochen lang nicht mit Antidepressiva therapiert worden ist.

2. Pharmazeutische Zusammensetzung zur Verwendung nach Anspruch 1, wobei es sich bei dem Modafinil um das linksdrehende Isomer von Modafinil handelt.

3. Pharmazeutische Zusammensetzung zur Verwendung nach Anspruch 1 oder Anspruch 2, wobei das Antidepressivum aus der Gruppe bestehend aus Trizyklika, selektiven Serotoninwiederaufnahmehemmern, Serotonin- und Noradrenalinwiederaufnahmehemmern, Monoaminoxidasehemmern und reversiblen Hemmern von Monoaminoxidase Typ A ausgewählt ist.

4. Pharmazeutische Zusammensetzung zur Verwendung nach Anspruch 3, wobei es sich bei dem Antidepressivum um Citalopram, Fluoxetin, Fluoxetinhydrochlorid, Paroxetin, Paroxetinhydrochlorid oder Clomipraminhydrochlorid handelt.

5. Pharmazeutische Zusammensetzung zur Verwendung nach Anspruch 4, wobei es sich bei dem Antidepressivum um Citalopram, Fluoxetin oder Fluoxetinhydrochlorid handelt.

6. Pharmazeutische Zusammensetzung zur Verwendung nach Anspruch 3, wobei es sich bei dem Antidepressivum um Venlafaxinhydrochlorid handelt.

7. Pharmazeutische Zusammensetzung zur Verwendung nach einem der Ansprüche 1-6, wobei das Modafinil in einer Menge von 50, 75, 100 oder 200 mg pro Tag verabreicht wird.

8. Pharmazeutische Zusammensetzung zur Verwendung nach einem der Ansprüche 1-6, wobei das Modafinil in einer Menge von 100 mg pro Tag verabreicht wird.

9. Pharmazeutische Zusammensetzung zur Verwendung nach einem der Ansprüche 1-8, wobei das Modafinil und das Antidepressivum in einem Gewichtsverhältnis von 1:1 bis 10:1 verabreicht werden.

10. Pharmazeutische Zusammensetzung zur Verwendung nach Anspruch 9, wobei das Modafinil und das Antidepressivum in einem Gewichtsverhältnis von 1:1 bis 7:1 verabreicht werden.

11. Pharmazeutische Zusammensetzung zur Verwendung nach Anspruch 10, wobei das Modafinil und das Antidepressivum in einem Gewichtsverhältnis von 1:1 bis 5:1 verabreicht werden.

12. Pharmazeutische Zusammensetzung zur Verwendung nach Anspruch 7, wobei das Antidepressivum in einer Menge von 10, 20, 30, 40 oder 50 mg pro Tag verabreicht wird.

## Revendications

1. Composition pharmaceutique comprenant le modafinil et un antidépresseur, destinée à être utilisée dans le traitement d'un trouble dépressif chez un patient, le patient n'ayant pas suivi de thérapie antidépressive pendant au moins 4 semaines.

2. Composition pharmaceutique destinée à être utilisée selon la revendication 1, **caractérisée en ce que** le modafinil est l'isomère lévogyre du modafinil.

3. Composition pharmaceutique destinée à être utilisée selon la revendication 1 ou la revendication 2, **caractérisée en ce que** l'antidépresseur est choisi dans le groupe constitué par les tricycliques, les inhibiteurs sélectifs de la recapture de la sérotonine, les inhibiteurs de la recapture de la sérotonine et de la noradrénaline, les inhibiteurs de la monoamine oxydase, et les inhibiteurs réversibles de la monoamine oxydase de type A.

4. Composition pharmaceutique destinée à être utilisée selon la revendication 3, **caractérisée en ce que** l'antidépresseur est le citalopram, la fluoxétine, le chlorhydrate de fluoxétine, la paroxétine, le chlorhydrate de paroxétine, ou le chlorhydrate de clomipramine.

5. Composition pharmaceutique destinée à être utilisée selon la revendication 4, **caractérisée en ce que** l'antidépresseur est le citalopram, la fluoxétine ou le chlorhydrate de fluoxétine.

6. Composition pharmaceutique destinée à être utilisée selon la revendication 3, **caractérisée en ce que** l'antidépresseur est le chlorhydrate de venlafaxine.

7. Composition pharmaceutique destinée à être utilisée selon l'une quelconque des revendications 1 à 6, **caractérisée en ce que** le modafinil est administré dans une quantité de 50, 75, 100 ou 200 mg par jour.

8. Composition pharmaceutique destinée à être utilisée selon l'une quelconque des revendications 1 à 6, **caractérisée en ce que** le modafinil est administré dans une quantité de 100 mg par jour.

9. Composition pharmaceutique destinée à être utilisée selon l'une quelconque des revendications 1 à 8, **caractérisée en ce que** le modafinil et l'antidépresseur sont administrés dans un rapport de 1:1 à 10:1, en poids.

10. Composition pharmaceutique destinée à être utilisée selon la revendication 9, **caractérisée en ce que** le modafinil et l'antidépresseur sont administrés dans un rapport de 1:1 à 7:1, en poids.

11. Composition pharmaceutique destinée à être utilisée selon la revendication 10, **caractérisée en ce que** le modafinil et l'antidépresseur sont administrés dans un rapport de 1:1 à 5:1, en poids.

12. Composition pharmaceutique destinée à être utilisée selon la revendication 7, **caractérisée en ce que** l'antidépresseur est administré dans une quantité de 10, 20, 30, 40 ou 50 mg par jour.
